Europäisches Patentamt

European Patent Office

Office européen des brevets

(11) Publication number: **0 221 317**
**B1**

(12) **EUROPEAN PATENT SPECIFICATION**

(45) Date of publication of patent specification: **05.12.90**

(51) Int. Cl.⁵: **C 12 N 15/75** // C12N15:68

(21) Application number: **86113091.2**

(22) Date of filing: **23.09.86**

(54) **Plasmid autonomously replicating at high temperatures.**

(30) Priority: **27.09.85 JP 212222/85**

(43) Date of publication of application:
**13.05.87 Bulletin 87/20**

(45) Publication of the grant of the patent:
**05.12.90 Bulletin 90/49**

(84) Designated Contracting States:
**DE FR GB**

(56) References cited:
**EP-A-0 034 470      EP-A-0 124 076**
**EP-A-0 057 976      EP-A-0 134 048**
**EP-A-0 060 663      EP-A-0 176 971**
**EP-A-0 096 586**

**Journal of Bacteriology, vol. 149, no. 3, March
1982, (Washington DC). T. Imanaka et al.
"Transformation of Bacillus stearothermophilus
with Plasmid DNA and Characterization of
Shuttle Vector Plasmids Between Bacillus
stearothermophilus and Bacillus subtilis", pages
824-830**

(73) Proprietor: **Sanraku Incorporated
15-1, Kyobashi 1-chome Chuo-ku
Tokyo (JP)**

(72) Inventor: **Aiba, Shuichi
31, Yamada-nishi 3-chome
Suita-shi Osaka-fu (JP)**
Inventor: **Koizumi, Jun-ichi
Morota-shukusha No. 3, 831-1
Nishikawatsu-cho Matsue-shi Shimane-ken (JP)**

(74) Representative: **Kraus, Walter, Dr. et al
Patentanwälte Kraus, Weisert & Partner
Thomas-Wimmer-Ring 15
D-8000 München 22 (DE)**

# EP 0 221 317 B1

**Description**

This invention relates to a vector plasmid which can be used suitably for thermophilic bacteria.

A host-vector system which can be used at relatively high fermentation temperatures has recently been energetically studied and developed because handling safety, high rates of replication and a reduction in cooling costs during fermentation can be expected.

Known vectors having such properties include, for example autonomously replicable recombinant plasmids pTB51 and pTB90 from thermophilic bacteria of the genus *Bacillus* (see Japanese Laid-Open Patent Publication No. 196092/1984), and plasmids pNHS171 and pNHK101 extracted from highly thermophilic bacteria of the genus *Thermus* isolated from spring water (see Japanese Patent Publications Nos. 8115/1985 and 53836/1984).

As stated above, some vector plasmids that can be used for thermophilic bacteria have been proposed heretofore. In particular, the recombinant plasmid pTB90 is suitable as a vector for *Bacillus stearothermophilus* which is extensively used as a thermophilic host organism and for production of various enzymes. This plasmid, however, has the defect that it does not always show stable autonomous replicability in the host organism, *Bacillus stearothermophilus,* at the optimum growth temperature (about 62°C) of the host organism or temperatures around it.

The present inventors have extensively worked from a multiplicity of standpoints in order to remove this defect, and have found that when a transformant obtained by transforming the aforesaid thermophilic bacterium with a recombinant plasmid derived from plasmid pTB90 is subjected to an ordinary mutating means, a certain desired mutation can be induced in the plasmid.

The "certain desired mutation", as used herein, means the phenomenon whereby the mutation treatment causes the plasmid to be integrated in host chromosomes, and by culturing the mutated organism under suitable conditions, the plasmid is excised from the chromosomes with the DNA sequence of the chromosome also excised joining the plasmid.

It has now been discovered that in the above phenomenon, the chromosomal DNA sequence excised joining the plasmid contains a gene which governs the function of replicating host cells at high temperatures and stabilizing their replication. This discovery has led to the present invention.

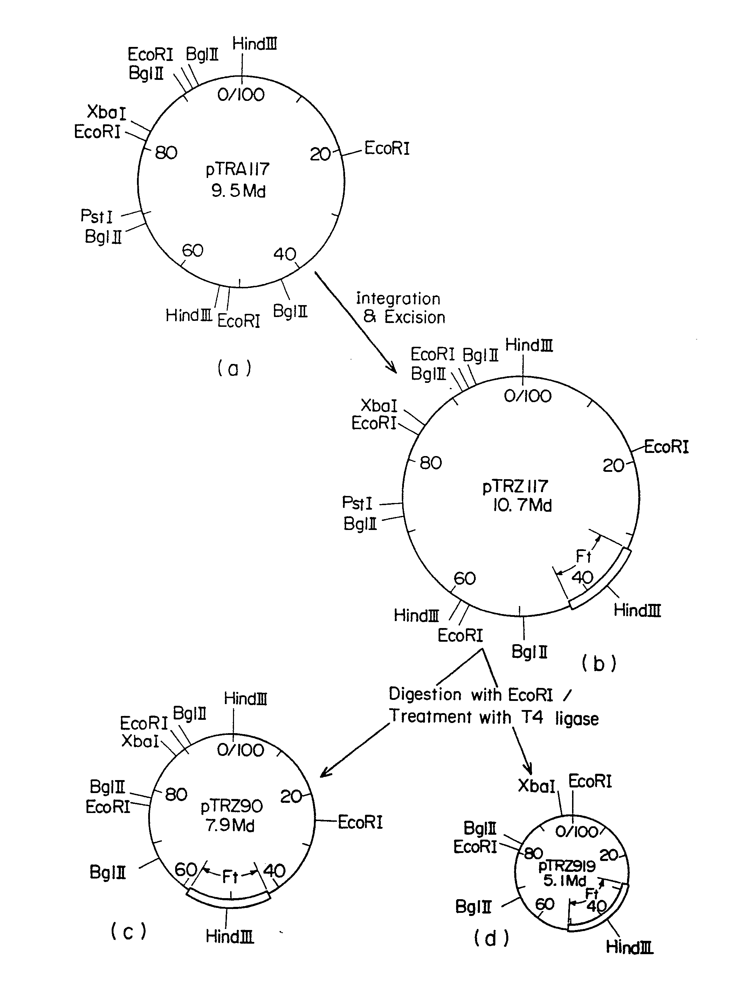

Thus, according to this invention, there is provided a vector plasmid (a molecular size of about 10.7 megadaltons) which contains a DNA sequence derived from chromosome of Bacillus stearothermophilus and which is characterized by restriction endonuclease map (b) shown in the accompanying drawing and which is obtainable from the host cells which have been deposited under FERM BP—1049 in accordance with the Budapest Treaty. Said plasmid contains a gene governing the function of replicating the host cells and stabilizing their replication.

Since this plasmid contains a gene which governs the function of replicating the host cells and stabilizing their replication, it can autonomously replicate stably in, for example, *Bacillus stearothermophilus,* a highly versatile bacterium, at its optimum growth temperature or temperatures around it.

The "gene governing the function of replicating host cells at high temperatures and stabilizing their replication" (to be referred to as "Ft gene") may be any of those derived from various microorganisms irrespective of their origins, or may be those prepared by chemical synthetic means. Since highly versatile *Bacillus stearothermophilus* is used as the host organism, a gene derived from *Bacillus stearothermophilus* is preferred. Specific examples of microorganism which can be sources of the above gene include bacteria of the genus *Bacillus* deposited as ATCC 12980, 10194, 8005, 7954 and 7953 in American Type Culture Collection, and mutants thereof such as CU21 strain derived from ATCC 12980 (for details of the characteristics of this strain, reference may be made to Imanaka et al., Journal of Bacteriology, vol. *149*, No. 3, page 825, 1982).

As shown by fragment Ft in plasmids pTRZ117, pTRZ90 and pTRZ919 in the accompanying drawing which is a flow diagram for constructing the vector plasmid of this invention containing a gene (Ft) which governs the function of replicating host cells at high temperatures and stabilizing their replication, the Ft gene is characterized by being included in a DNA sequence having a molecular size of about 1.2 MD and only one site of cleavage with restriction endonuclease *Hind*III.

So long as the vector plasmid provided by this invention comprises the aforesaid DNA sequence containing the Ft gene, its other DNA sequence is not at all restricted. Thus, so long as the vector plasmid of the invention contains the DNA sequence containing the Ft gene, it may have another DNA sequence which is suitable depending upon the host used, the purpose of its use, etc. However, in using the thermophilic bacterium, *Bacillus stearothermophilus,* as the host, the Ft gene is preferably joined to a constituent DNA sequence of the vector plasmid which can autonomously replicate stably within the host.

Specific preferred examples of such constituent DNA sequence are the recombinant plasmids pTRZ117, pTRZ90 and pTRZ919 characterized by the restriction endonuclease maps (b), (c) and (d) shown in the attached drawing.

In the restriction endonuclease maps, represents the above gene fragment having a molecular size of 1.2 Md excised from a chromosome.

2

The method of constructing the vector plasmid of this invention will now be described.

(a) The thermophilic bacterium mentioned above is used as a host and transformed by a known protoplast method with a plasmid having a suitable selection marker and being capable of transforming the host. Specific examples of the host organism are *Bacillus stearothermophilus* ATCC 12980, 10194, 8005, 7954 and 7953, and mutants thereof such as Cu21.

Specific examples of the plasmid used in transformation include recombinant plasmids obtained by attaching a selection marker to pTB51, pTB52, pTB53 and pTB90 constructed from plasmid pTB19 or pTB20 (see Journal of Bacteriology, vol. 149, p. 824—830 cited above). For example, pLP11 (9.5 Md) obtained by inserting a DNA fragment (2.8 Md) containing a penicillinase gene *penP* into pTB90 [Journal of General Microbiology, *128*, 2997—3000 ([1982)] may be used. This recombinant plasmid may be prepared by a known gene recombinant operation using plasmid pTTE11 containing the *penP* gene fragment [Journal of Bacteriology, *147*, No. 3, pp. 775—786 (1981)] and pTB90 [Molecular Cloning, A Laboratory Manual, Cold Spring Harbor Laboratory, Cold Spring Harbor, N.Y., 1982.

(b) The transformant obtained by the method (a) is subjected to a mutation treatment by a known mutating means using a mutagen such as N-mehyl-N'-nitro-N-nitrosoguanidine (to be referred to as NTG). From the mutants prepared by using various selection markers as indices, plasmid-bearing mutants in which the plasmid exists in the cytoplasm in cultivation at about 48°C, but does not in cultivation at about 63°C are selected.

(c) Mutated plasmids are collected from the mutants obtained and selected in (b) by a method known *per se* (see the above-cited Molecular Cloning). A specific example of the mutated plasmid is plasmid pTRA117 which can be extracted by a known plasmid extracting method (see the above-cited Molecular Cloning) from a strain deposited under FERM P—8450 on September 25, 1985 at Fermentation Research Institute, Agency of Industrial Science and Technology, 1—3, Higashi, 1-chome, Yatabe-machi, Tsukubagun, Ibaraki-ken, Japan and transferred on May 25, 1986 to international deposition FERM BP—1048 under the Treaty on the International Recognition of the Deposit of Microorganisms for the Purposes of Patent Procedure. A thermophilic bacterium having on its chromosome a gene which governs the function of replicating host cells and stabilizing their replication is transformed with this mutated plasmid by using the protoplast method.

(d) The transformants harboring the mutated plasmid obtained in (c) are inoculated in a nutrient medium and cultured overnight at about 48°C. The culture fluid is inoculated in the same medium, and cultured for several hours at about 63°C to integrate the mutated plasmid into the host chromosomes. The resulting cells are inoculated in the above nutrient medium and cultured overnight at about 48°C. As a result of this culturing, the Ft gene which governs the function of replicating host cells at high temperatures and stabilizing their replication and which is derived from the host chromosomes is excised from the chromosome and joined to the mutated plasmid to form a new plasmid within the cells. A specific example of this plasmid is plasmid pTRZ117 [see the restriction endonuclease map (b) in the accompanying drawing] extracted by the aforesaid method from the strain deposited under FERM P—8456 by the present Applicant on September 25, 1985 at the aforesaid depository and transferred on May 15, 1986 to international deposit under FERM BP—1049 in accordance with the Budapest Treaty.

The plasmid of this invention can achieve the object of this invention if only it comprises a DNA sequence containing the Ft gene which is excised from the chromosomal DNA by the above method and governs the function of replicating host cells at high temperatures and stabilizing their replication, and the other DNA sequence of the plasmid is not particularly limited. Accordingly, a plasmid containing the Ft gene which is derived by, for example, treating the above pTRZ117 by an *in vivo* or *in vitro* technique is also included within the plasmid of this invention. Examples of the plasmid derived by such a treatment are plasmids pTRZ90 and pTRZ919 [see the restriction endonuclease maps (c) and (d) in the accompanying drawing] smaller than pTRZ117 which are obtained by a known treating method, for example by digesting pTRZ117 with restriction endonuclease *Eco*RI and treating the digestion product with T4-ligase.

The vector plasmid of this invention is useful as a vector having the function of replicating host cells at high temperatures and stabilizing their replication presumably because it comprises a gene fragment considered to govern the function of replicating a thermophilic bacterium at high temperatures and stabilizing its replication.

The present invention will be illustrated below by an example in which *Bacillus stearothermophilus* (Cu21 (ATCC 12980) is used as a host organism and the plasmid pLP11 (a gene of a kanamycin-inactivating enzyme having *Ran* and a penicillinase gene *penP*) is used as a recombinant plasmid.

(A) *B. stearothermophilus* (ATCC 12980) was transformed with pLP11 by the conventional protoplast method. The transformant was suspended in a NTG solution (10 micrograms/ml) and contacted with NTG for 15 minutes.

The cells which experienced contact with NTG were developed on an L-agar medium containing 500 micrograms/ml of kanamycin and cultured overnight at 60°C.

Colonies grown and formed on the agar medium were replicated on an L-agar medium containing 500 micrograms/ml of kanamycin and 0.75% (w/v) of polyvinyl alconol, and cultured overnight at 48°C to form colonies again. Thereafter, the agar medium was stained with a solution of 0.8 N I, 0.32 N KI and 1% (w/v) $NaB_4O_7$. A penicillin solution (20,000 U/ml) was developed on the stained agar medium. Those colonies around which no halo appeared were discarded. The cells in the remaining colonies were cultured at 63°C, and those colonies which did not show replication were discarded.

The colonies which showed replication were cultured overnight at 48°C in an L medium containing 100 micrograms/ml of kanamycin, and plasmids were isolated from these colonies. The CU 21 strain was transformed using the isolated plasmids.

The transformants obtained with the candidate plasmids were inoculated in an L-agar medium containing 500 picograms/ml of kanamycin, and cultured overnight at 60°C, and those candicate plasmids which gave transformants incapable of colony formation were excluded.

Transformants which showed colony formation were further cultured in an L-agar medium containing 500 micrograms/ml of kanamycin and 0.75% (w/v) of polyvinyl alcohol and in an L-medium containing 100 micrograms/ml of kanamycin. Those candidate plasmids which have transformants incapable of meeting the selection standards were discarded.

Transformants obtained by using the remaining candidate plasmids were cultivated at 48 and 63°C respectively in an L-medium containing 100 micrograms/ml of kanamycin, and plasmid-bearing transformants having such a property that in cultivation at 48°C, a plasmid existed in the cytoplasm but in cultivation at 63°C, no plasmid existed in the cytoplasm were screened and selected. At this time, the plasmids were extracted from the cultivated cells by a method capable of extracting only a plasmid existing in the state of a cytoplasm factor from the cultured cells (alkali extracting method; see the above-cited Molecular Cloning).

One of the plasmids extracted from the selected cultivated cells was named pTRA11.

(B)  B. stearothermophilus CU21 was again transformed with pTRA117. The resulting transformants were inoculated in an L-medium containing 100 micrograms/ml of kanamycin and cultured at 63°C for 6 hours to integrate pTRA117 into the host chromosomes.

The cells resulting from integration of pTRA117 into the host chromosomes were inoculated in a medium of the same composition as above were cultured overnight at 48°C. By this procedure, pTRA117 integrated into the chromosomes was again excised from the chromosomes. At this time, pTGRA117 to which the chromosomal DNA fragment was joined was excised from the chromosomes (cytoplasm). At the time of excision, the chromosomal DNA fragment and pTRA117 formed a new plasmid.

The culture fluid was inoculated in a medium having the same composition, and cultivated at 65°C, and the replicated cells were selected.

The plasmid pTRZ117 (10.7 Md) obtained from the cultivated cells had a DNA sequence (1.2 mD) containing a gene which is derived from the chromosomes of the host organism CU21 strain and governs the function of replicating the host cells at high temperatures and stabilizing their replication and having a molecular size of 1.2 Md and only one site of cleavage with restriction endonuclease HindIII.

(C)  Construction of plasmids pTRZ90 and pTRZ919

In accordance with the above-cited Manual, pTRZ117 was digested with EcoRI and treated with T4-ligase to construct plasmids pTRZ90 and pTRZ919 which were smaller than pTRZ117. Transformants of B. stearothermophilus harboring these plasmids could also be replicated at 65°C.

(D)  Southern hybridization described below led to the determination that the 1.2Md DNA fragment newly included in the plasmids of the pTRZ series was derived from chromosomes.

An EcoR digestion product of pTRZ919 was subjected to low-melting garose gel electrophoresis, and a 4.1 Md EcoRI fragment containing a 1.2Md DNA fragment [the fragment shown by Ft in the restriction endonuclease maps (b), (c) and (d) in the accompanying drawing] was separated and recovered. The resulting fragment was labelled with $^{32}P$ by the nick translation method to prepare a probe.

It could be confirmed by this that the chromosomes of the host organism, B. stearothermophilus, hybridizes with the probe.

The L-medium used in the above procedure had the following composition.

Composition of the L-medium

| Bactotrypton | 10 g/liter |
|---|---|
| Yeast extract | 5 g/liter |
| NaCl | 5 g/liter |

Effect

Transformants obtained with the plasmids indicated below were cultured in an L-medium containing 100 micrograms/ml of kanamycin for 5 to 10 hours at the temperatures indicated in the following table. The effects of the temperatures on the replication of the transformants in the L-medium are shown in the following table.

| | Temperature ($^{\circ}$C) | | | | | |
|---|---|---|---|---|---|---|
| Plasmid | 48 | 55 | 59 | 63 | 65 | 67 |
| pTB90 | + | + | $\pm$ | - | - | - |
| pTRZ117 | + | + | + | + | + | - |
| pTRZ90 | + | + | + | + | + | - |
| pTRZ919 | + | | | | + | - |
| pTRA117 | + | + | + | + | - | - |

The scale of evaluation was as follows:

+: showing such replication that the cell concentration is at least about 0.1 mg/liter

−: showing no replication

±: the same replication as + could be determined by culturing for 20 hours.

As stated above, it could be confirmed that transformants obtained with the plasmids of this invention (plasmids pTRZ117, pTRZ90 and pTRZ919) could autonomously replicate stably even at temperatures above the optimum growth temperature of *B. stearothermophilus.*

## Claims

1. A vector plasmid (a molecular size of about 10.7 megadaltons) which contains a DNA sequence derived from chromosome of Bacillus stearothermophilus and which is characterized by restriction endonuclease map (b) shown in the accompanying drawing and which is obtainable from the host cells which have been deposited under FERM BP—1049 in accordance with the Budapest Treaty.

2. A vector plasmid (a molecular size of about 7.9 megadaltons) which is derived from the vector plasmid of Claim 1 and is obtained by the deletion from said vector of the 2.8 Md Eco RI fragment containing unique Hind III, Bgl II and Pst I restriction sites and which is characterized by restriction endonuclease map (c) shown in the accompanying drawing.

3. A vector plasmid (a molecular size of about 5.1 megadaltons) which is obtained from the vector plasmid of Claim 1 by the deletion of the 2.8 Md Eco RI fragment according to claim 2 and the deletion of another Eco RI fragment of the same size containing a Bgl II and a Hind III restriction site and which is characterized by restriction endonuclease map (d) shown in the accompanying drawing.

## Patentansprüche

1. Vektorplasmid (Molekulargröße von etwa 10,7 Megadalton), das eine DNS-Sequenz, die von dem Chromosom von Bazillus Stearothermophilus abgeleitet ist, enthält dadurch gekennzeichnet, daß es die Restriktionskarte (b), die in der beigefügten Zeichnung gezeigt ist, aufweist, und daß es aus Wirtzellen, die unter FERM BP—1049, gemäß dem Budapester Übereinkommen, hinterlegt wurden, erhalten werden kann.

2. Vektorplasmid (Molekulargröße von etwa 7,9 Megadalton), dadurch gekennzeichnet, daß es sich von dem Vektorplasmid nach Anspruch 1 ableitet und durch Deletion des 2,8 Md Eco RI Fragments, das einzig die Hind III, BGL II und Pst I Restriktionsschnittstellen enthält, aus diesem Vektor erhalten wird und daß ea durch die Restriktionskarte (c), die in der beigefügten Zeichnung gezeigt ist, gekennzeichnet ist.

3. Vektorplasmid (Molekulargröße von etwa 5,1 Megadalton), dadurch gekennzeichnet, daß es aus dem Vektorplasmid nach Anspruch 1 durch Deletion des 2,8 Md Eco RI Fragments nach Anspruch 2 und durch Deletion eines weiteren Eco RI Fragments der gleichen Größe, das eine BGL II und eine Hind III Restriktionsschnittstelle enthält, erhalten wird und daß es durch die Restriktionskarte (d), die in der beigefügten Zeichnung gezeigt, ist, charakterisiert ist.

## Revendications

1. Plasmide vecteur (d'une taille moléculaire d'environ (10,7 mégadaltons) qui contient une séquence d'ADN provenant du chromosome de *Bacillus stearothermophilus*, qui est caractérisé par la carte d'endonucléases de restriction (b) figurant sur le dessin annexé et qui peut être obtenu à partir des cellules-hôtes qui ont été déposées sous la référence FERM BP—1049 conformément au Traité de Budapest.

2. Plasmide vecteur (d'une taille moléculaire d'environ (7,9 mégadaltons) qui est dérivé du plasmide vecteur de la revendication 1 et obtenu par la délétion dans ledit vecteur du fragment *Eco*RI de 2,8 Md contenant des sites uniques de restriction *Hin*dIII, *BG*II et *Pst*I et qui est caractérisé par la carte d'endonucléases de restriction (c) figurant sur le dessin annexé.

3. Plasmide vecteur (d'une taille moléculaire d'environ (5,1 mégadaltons) qui est obtenu à partir du plasmide vecteur de la revendication 1 par la délétion du fragment *Eco*RI de 2,8 Md selon la revendication 2 et la délétion d'un autre fragment *Eco*RI de la même taille contenant un site de restriction *Bg*II et un site de restriction *Hin*dIII et qui est caractérisé par la carte d'endonucléases de restriction (d) figurant sur le dessin annexé.

EcoRI BglⅡ
BglⅡ ⟍⟍     HindⅢ
XbaI ⟍      0/100
EcoRI ⟍ 80
            pTRA117
            9.5 Md            20 ⟋ EcoRI
PstI ⟋
BglⅡ ⟋ 60        40
HindⅢ EcoRI   BglⅡ

( a )

integration
& Excision

EcoRI BglⅡ   HindⅢ
BglⅡ ⟍⟍      0/100
XbaI ⟍
EcoRI ⟍ 80
                            EcoRI
            pTRZ117       20
            10.7 Md
PstI ⟋
BglⅡ ⟋                Ft
                         40
            60              HindⅢ
HindⅢ
EcoRI    BglⅡ          ( b )

Digestion with EcoRI /
Treatment with T4 ligase

HindⅢ
EcoRI BglⅡ    0/100
XbaI
BglⅡ ⟍ 80
EcoRI ⟍        pTRZ90    20
               7.9 Md           EcoRI
BglⅡ ⟋ 60      Ft   40
                HindⅢ
( c )

XbaI  EcoRI
          0/100
BglⅡ
EcoRI ⟍ 80
         pTRZ919   20
         5.1 Md   Ft
BglⅡ    60   40
            HindⅢ
( d )

1